# EUROPÄISCHE PATENTSCHRIFT

(11) **EP 1 272 187 B1**
(45) Veröffentlichungstag und Bekanntmachung des Hinweises auf die Patenterteilung: **30.07.2008**
(21) Anmeldenummer: 01923699.1
(22) Anmeldetag: 26.03.2001
(51) Int. Cl.: A61K 31/505, A61K 31/495, A61P 25/30, A61P 25/32, A61P 25/34, A61P 25/36, A61P 43/00

(54) **DOPAMIN-D3-REZEPTOR-LIGANDEN ZUR BEHANDLUNG VON SUCHT**
DOPAMINE-D3 RECEPTOR LIGANDS FOR THE TREATMENT OF ADDICTION
LIGANDS DU RECEPTEUR D3 DE LA DOPAMINE DESTINES AU TRAITEMENT DE LA DEPENDANCE

(30) Priorität: 27.03.2000 DE 10015211
(43) Veröffentlichungstag der Anmeldung: 08.01.2003
(73) Patentinhaber: Abbott GmbH & Co. KG, 65205 Wiesbaden (DE)
(72) Erfinder: STARCK, Dorothea, 67059 Ludwigshafen (DE); TREIBER, Hans, Jörg, 68782 Brühl (DE); UNGER, Liliane, 67065 Ludwigshafen (DE); TESCHENDORF, Hans-Jürgen, 67373 Dudenhofen (DE); GROSS, Gerhard, 67346 Speyer (DE)
(74) Vertreter: Kinzebach, Werner
(86) Internationale Anmeldenummer: PCT/EP2001/003411
(87) Internationale Veröffentlichungsnummer: WO 2001/072306

(56) Entgegenhaltungen:
- EP-A- 0 779 284
- WO-A-01/49679
- WO-A-92/22542
- WO-A-95/25727
- WO-A-96/02246
- WO-A-96/02249
- WO-A-96/02519
- WO-A-96/02520
- WO-A-99/02503

## Beschreibung

Die vorliegende Erfindung betrifft die Verwendung bestimmter Dopamin-D₃-Rezeptor-Liganden zur Behandlung von Erkrankungen des Zentralen Nervensystems, vor allem die Behandlung psychischer Störungen, die durch psychotrope Substanzen vermittelt werden.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Störungen im dopaminergen Transmittersystem resultieren in Erkrankungen, wie z.B. Schizophrenie, Depression und Parkinson'sche Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514).

Mittlerweile teilt man die Dopamin-Rezeptoren in zwei Familien ein. Einerseits die D₂-Gruppe bestehend aus D₂-, D₃- und D₄-Rezeptoren, andererseits die D₁-Gruppe bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Selektive Dopamin-D₃-Rezeptor-Liganden sind bekannt. Beispielsweise wird in Dubuffet et al. (1999) Bioorg. Med. Chem. Lett. 9, 2059-2064, eine Substanz der Formel 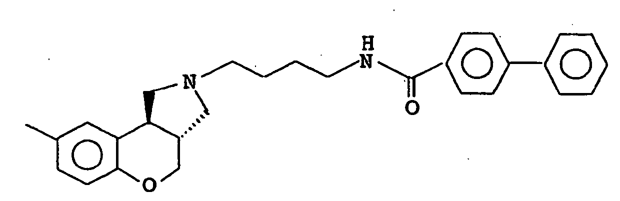 oder in EP 779 284 sowie Pilla et al. (1999) Nature 400, 371-375 eine Substanz der Formel 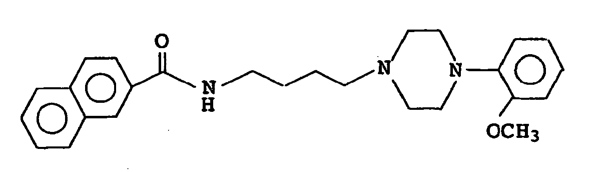 genannt. Weitere Arylpiperazine mit selektiver Affinität zu Dopamin-D₃-Rezeptoren werden von P.J. Murray et al. in Bioorganic & Medicinal Chemistry Letters, Vol. 5, No. 3, 219-222 (1995) beschrieben.

Weitere Verbindungen zur Behandlung von Erkrankungen, die auf Dopamin-D₃-Liganden ansprechen, sind aus der WO 96/02519; WO 96/02520; WO 96/02249; WO 96/02246; WO 97/25324; WO 99/02503 und WO 98/05178 bekannt. Bekanntermaßen sind diese Verbindungen zur Behandlung von Schizophrenie, Depressionen, Neurosen und Psychosen brauchbar.

Nach wie vor besteht ein erheblicher Bedarf an effektiven Behandlungsmöglichkeiten diverser Erkrankungen des Zentralen Nervensystems. Beispielsweise kann Suchtphänomenen und damit in Zusammenhang stehenden Störungen der Psyche und des Verhaltens drogen-oder arzneimittelabhängiger Individuen in vielen Fällen bisher nur unzureichend begegnet werden. Während Heroinabhängigkeit wohl mit Opioid-Agonisten, z.B. Methadon, behandelt werden kann, gibt es gegen Kokainmißbrauch zur Zeit noch keine Pharmakotherapie.

Überraschenderweise wurde nun gefunden, dass bestimmte Verbindungen ein geeignetes pharmakologisches Wirkungsprofil aufweisen, so dass sie zur Behandlung diverser Erkrankungen des Zentralen Nervensystems eingesetzt werden können.

Gegenstand der vorliegenden Erfindung ist daher die Verwendung wenigstens einer Verbindung der allgemeinen Formel I

L - D - B - G (I)

worin
- L: für einen 5- oder 6-gliedrigen aromatischen Heteromonocyclus L1 mit 1, 2 oder 3 unter O, N und S unabhängig voneinander ausgewählten Heteroatomen
oder einen aromatischen oder heteroaromatischen unter der Gruppe L2 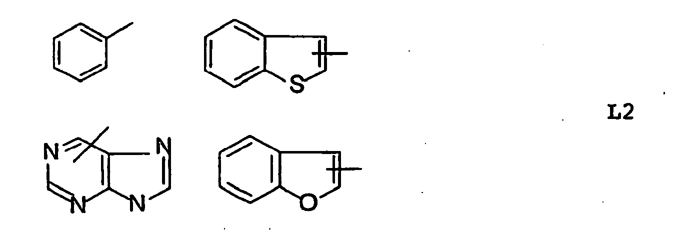 ausgewählten Rest steht, wobei L gegebenenfalls 1, 2, 3 oder 4 Substituenten aufweist, die unabhängig voneinander ausgewählt sind unter C₁-C₈-Alkyl, das gegebenenfalls durch OH, OC₁-C₈-Alkyl, Phenyl oder Halogen substituiert ist; OR¹, C₂-C₆-Alkenyl, C₂-C₆-Alkinyl, C₃-C₈-Cycloalkyl, Halogen, CN, CONR¹R², COOR¹, NO₂, NR¹R², SR¹, SO₂R¹, SO₂NR¹R², OSO₂R¹, Ax1 oder Phenoxy, das gegebenenfalls durch C₁-C₆-Alkyl, OC₁-C₆-Alkyl oder Halogen substituiert ist; C₁-C₆-Alkanoyl oder Benzoyl;
worin
- Ax1: für Phenyl, Naphthyl oder einen 5- oder 6-gliedrigen heterocyclischen aromatischen Ring mit 1, 2, 3 oder 4 Heteroatomen steht,
wobei Ax1 gegebenenfalls 1, 2, 3 oder 4 Substituenten aufweist, die unabhängig voneinander ausgewählt sind unter C₁-C₆-Alkyl, das gegebenenfalls durch OH, OC₁-C₆-Alkyl, Halogen oder Phenyl substituiert ist; C₁-C₆-Alkoxy, C₂-C₆-Alkenyl, C₂-C₆-Alkinyl, C₃-C₆-Cycloalkyl, Halogen, CN, COR¹, COOR¹, NR¹R², NO₂, SR¹, SO₂R¹, SO₂NR¹R², oder Phenyl, das gegebenenfalls durch C₁-C₆-Alkyl, OC₁-C₆-Alkyl, NR¹R², CN, CF₃, CHF₂, oder Halogen substituiert ist; und wobei der erwähnte heterocyclische aromatische Ring gegebenenfalls mit einem Phenylring kondensiert sein kann;
- R¹: für H, C₃-C₆-Cycloalkyl, Phenyl oder C₁-C₉-Alkyl, das gegebenenfalls durch OH, OC₁-C₈-Alkyl, Halogen oder Phenyl substituiert ist, steht;
- R²: die für R¹ angegebenen Bedeutungen besitzt oder für COR¹ oder CO₂R¹ steht;
- D: für eine C₁-C₁₈-Alkylengruppe oder eine C₁-C₁₈-Alkylengruppe, die wenigstens eine Gruppe Z umfaßt, die ausgewählt ist unter O S, NR², C₃-C₆-Cycloalkyl, CO, CONR², CH₂, einer Doppel- und einer Dreifachbindung, wobei R² wie oben definiert ist, steht;
- B: für einen 6-, 7- oder 8-gliedrigen gesättigten Ring mit einem oder zwei Stickstoffheteroatomen steht, wobei sich die Stickstoffheteroatome in 1,4- oder 1,5-Position befinden und der Ring in 1-Position an den Rest D und in 4oder 5-Position an den Rest G gebunden ist und wobei der Ring außerdem eine Doppelbindung in 3- oder 4-Position aufweisen kann;
- G: für Phenyl, Pyridyl, Pyrimidinyl oder Triazinyl steht, wobei G gegebenenfalls 1, 2, 3 oder 4 Substituenten aufweisen kann, die unabhängig voneinander ausgewählt sind unter OR¹, C₁-C₈-Alkyl, C₂-C₈-Alkenyl, C₂-C₈-Alkinyl, C₁-C₆-Alkoxy-C₁-C₆-alkyl, Halogen-C₁-C₆-alkyl, Halogen-C₁-C₆-alkoxy, Halogen, CN, CO₂R¹, NO₂, SO₂R¹, SO₃R¹, NR¹R², SO₂NR¹R², SR¹, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 oder 2 unter O, S und N unabhängig voneinander ausgewählten Heteroatomen, wobei der carbocyclische oder der heterocyclische Ring gegebenenfalls substituiert ist durch C₁-C₈-Alkyl, Phenyl, Phenoxy, Halogen, OC₁-C₈-Alkyl, OH, NO₂, oder CF₃,
wobei G gegebenenfalls mit einem carbocyclischen oder heterocyclischen Ring der oben definierten Art kondensiert sein kann;
die ausgewählt sind unter
sowie deren Salze mit physiologisch verträglichen Säuren,
zur Herstellung eines Medikaments zur Behandlung von Sucht.
2-[(3-{4-[2-tert-Butyl-6-(trifluormethyl)-4-pyrimidinyl]-1-piperazinyl}propyl)sulfanyl]-4-pyrimidinol,
5-({3-[4-(3,5-Dichlorphenyl)-1-piperazinyl]propyl}sulfanyl)-1,3,4-thiadiazol-2-amin,
5-[(2-Methyl-3-(4-[3-(trifluormethyl)phenyl]-1-piperazinyl)propyl)sulfanyl]-1,3,4-thiadiazol-2-amin,
2-[{3-(4-[3-(Trifluormethyl)phenyl]-1-piperazinyl}propyl)sulfanyl]-4-pyrimidinol,
5-(6-{4-[3-(Trifluormethyl)phenyl]-1-piperazinyl)hexyl)-1,3,4-thiadiazol-2-amin,
1-{(2E)-4-[(4-Hydroxy-2-pyrimidinyl)sulfanyl]-2-butenyl}-4-[3-(trifluormethyl)phenyl]piperazindiium-dichlorid,
4-Methyl-5-[(3-(4-[3-(trifluormethyl)phenyl]-1-piperazinyl)propyl)sulfanyl]-4H-1,2,4-triazol-3-amin,
2-([3-(4-[3-(Trifluormethyl)phenyl]-3,6-dihydro-1(2H)-pyridinyl)propyl]sulfanyl)-4-pyrimidinol,
2-({3-[4-(3-Isopropylphenyl)-1-piperazinyl]propyl}sulfanyl)-4-pyrimidinol,
5-[(3-{4-[6-(Trifluormethyl)-2-pyridinyl]-1-piperazinyl}propyl)sulfanyl}-1,3,4-thiadiazol-2-amin,
5-[((2E)-4-(4-[6-(Trifluormethyl)2-pyridinyl]-1-piperazinyl)-2-butenyl)sulfanyl]-1,3,4-thiadiazol-2-amin,
5-({3-[4-(3,5-Ditert-butylphenyl)1-piperazinyl]propyl}sulfanyl)-4-methyl-4H-1,2,4-triazol-3-amin,
5-({7-[4-(3-lsopropylphenyl)-1-piperazinyl]heptyl}sulfanyl)-1,3,4-thiadiazol-2-amin,
4-Ethyl-5-[(3-(4-[3-(trifluormethyl)phenyl]-1-piperazinyl)propyl)sulfanyl]-4H-1,2,4-triazol-3-amin,
3-(4-{8-[(5-Amino-1,3,4-thiadiazol-2-yl)sulfanyl]octyl)-1-piperazinyl)benzonitril,
5-[(3-(4-[6-Methyl-2-(1H-pyrrol-1-yl)-4-pyrimidinyl]-1-piperazinyl}propyl)sulfanyl]-1,3,4-thiadiazol-2-amin,
4-Methyl-5-[(3-{4-[6-Methyl-2-(1H-pyrrol-1-yl)-4-pyrimidinyl]-1-piperazinyl}propyl)sulfanyl]-4H-1,2,4-triazol-3-amin,
5-[{(2E)-4-(4-[2-tert-Butyl-6-(trifluormethyl)-4-pyrimidinyl]-1-piperazinyl)-2-methyl-2-butenyl}sulfanyl]-4-methyl-4H-1,2,4-triazol-3-amin,
1-[3-(Difluormethyl)phenyl)-4-(3-([4-methyl-5-(methylammonio)-4H-1,2,4-triazol-3-yl]sulfanyl)propyl)piperazin-4-ium-di-chlorid und
5-({3-[4-(2,6-Ditert-butyl-4-pyrimidinyl)-1-piperazinyl]propyl}sulfanyl)-1,3,4-thiadiazol-2-amin,

Der Begriff "Sucht" steht erfindungsgmäß für die Abhängigkeit eines Individuums von exogenen und/oder endogenen Reizen und/oder für eine Gewöhnung an exogene und/oder endogene Reize.

Die Abhängigkeit kann physischer und/oder psychischer Natur sein.

Eine physische Abhängigkeit kann sich insbesondere in einem Entzugssyndrom manifestieren. Ein Entzugsyndrom ist eine unerwünschte physiologische Veränderung, die beispielsweise dann eintritt, wenn die Intensität eines suchtvermittelnden Reizes verringert wird, bzw. dem Reiz entgegengewirkt wird und insbesondere der Reiz unterbunden wird.

Eine psychische Abhängigkeit kann insbesondere von einem Gefühl der Genugtuung und dem Wunsch, den Reiz zu wiederholen, begleitet sein.

Gewöhnung als Sucht kennzeichnendes Merkmal umschreibt den Umstand, die Intensität eines Reizes progressiv erhöhen zu müssen, um eine bestimmte Wirkung erzielen zu können.

Gemäß einem weiteren Aspekt der vorliegenden Erfindung steht der Begriff "Sucht" für Störungen der Psyche und des Verhaltens eines Individuums" die mit suchtvermittelnden Reizen in zusammenhang stehen. Hierzu gehören vor allem ein suchttypisches Verhalten, insbondere ein zwanghaftes Verhalten bzw. heftiges Verlangen (Craving) und/oder die überwiegende Ausrichtung individueller Aktivitäten auf das zuführen suchtvermittelnder Reize.

Erfindungsgemäß wird unterschieden zwischen Sucht, die durch exogene Faktoren ausgelöst ist, und Sucht als Folge endogener Faktoren.

Zu exogenen Faktoren gehören vor allem psychotrope Substanzen. Eine psychotrope Substanz im erfindungsgemäßen Sinne kann dadurch gekennzeichnet sein, daß sie in einem Organismus Gewöhnung, physische Abhängigkeit und/oder psychische Abhängigkeit bewirkt. Abhängigkeit im Zusammenhang mit der Verwendung psychotroper Substanzen kann auch mit dem Begriff Drogenabhängigkeit bezeichnet werden.

Zu Abhängigkeit bewirkenden Substanzen gehören insbesondere diejenigen, die auf das zentrale Nervensystem wirken. Wirkungen in diesem Sinne sind vor allem eine Verringerung von Angst und Anspannung, Veränderungen des Gemütszustandes, die vom Betroffenen als angenehm empfunden werden, z.B. gehobene Stimmung oder Euphorie, das Gefühl einer gesteigerten mentalen und/oder physischen Leistungsfähigkeit, eine veränderte sensorische Wahrnehmung und/oder verhaltensveränderungen.

Eine besondere Gruppe erfindungsgemäß zu behandelnder Suchterkrankungen sind gekennzeichnet durch ein Muster aus Gewöhnung und psychischer Abhängigkeit und in besonderen Fällen auch physischer Abhängigkeit.

Zu endogen Faktoren gehören insbesondere Störungen von Transmittersystemen, vor allem des dopaminergen Systems. So ist beispielsweise Spielsucht erfindungsgemäß behandelbar.

Psychotrope Substanzen werden in anderen Zusammenhängen als Gifte, insbesondere Genußgifte, Arzneimittel, Drogen oder Lösemittel bezeichnet. Zu diesen Substanzen gehören beispielsweise Stimulantien, wie Opioide, z.B. Morphin, Heroin und Codein; amphetaminartige Substanzen, z.B. Amphetamin, Methylphenidat und Kokain; Nikotin; Alkohol; Substanzen, die mit dem GABA-Chlorid-Kanal-Komplex interagieren; Sedativa; Anxiolytika, Hypnotika oder Tranquillizer, z.B. Benzodiazepine und Barbiturate; Halluzinogene, z.B. LSD; Cannabinoide, z.B. Marijuana; psychomotorische Stimulantien, wie 3,4-Methylen-dioxy-N-methylamphetamine (Ecstasy); Phencyclidin; oder sonstige Stimulantien, Coffein eingeschlossen. Im Rahmen der erfindungsgemäßen Behandlung sind vor allem Störungen in Betracht zu ziehen, die durch Opioide, insbesondere Codein, durch amphetaminartige Substanzen, insbesondere Kokain oder Amphetamin, Nikotin und Alkohol vermittelt sind.

Eine auf die Suchtbehandlung abzielende besondere Ausführungsform der vorliegenden Erfindung betrifft vor allem die Behandlung von Suchtsymptomen, wie Entzugserscheinungen, zwanghaftem Verhalten und heftigem Verlangen (Craving) im Hinblick auf die suchterzeugende Substanz(en). Ziel der erfindungsgemäßen Behandlung ist es insbesondere, die vom betroffenen Individuum empfundene Ausprägung und Stärke der Suchtsymptome zu verringern und vorzugsweise zu unterdrücken, so daß gemäß einem besonderen Aspekt der vorliegenden Erfindung die Suchtentwöhnung erleichtert bzw. die Rückfallhäufigkeit in die Sucht nach Abstinenz verringert wird. So betrifft die vorliegende Erfindung eine insbesondere auf die Einnahme und unter Umständen den Mißbrauch psychotroper Substanzen gerichtete Behandlung, wobei vor allem die Motivation zur Sucht verringert wird.

Gemäß einem weiteren Aspekt der vorliegenden Erfindung richtet sich die Behandlung auf Störungen, deren Ursachen zumindest zum Teil auf eine anomale Aktivität von Dopamin-D₃-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnern (Liganden) an Dopamin-D₃-Rezeptoren im Sinne einer zweckmäßigen medizinischen Behandlung beeinflussen lassen. Gemäß einer besonderen Ausführungsform werden diejenigen Störungen behandelt, die sich durch eine wenigstens partielle Aktivierung an Dopamin-D₃-Rezeptoren beeinflussen lassen. Hierzu gehören eine partielle und auch eine vollständige Agonisierung an Dopamin-D₃-Rezeptoren.

Erfindungsgemäß zu behandelnde Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäße Behandlung von Störungen des Zentralen Nervensystems lassen sich eine Vielzahl von Anzeichen, Symptomen und/oder Fehlfunktionen behandeln, die mit den Störungen und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlußkraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Erfindungsgemäß verwendet man zur Behandlung der vorstehend genannten Indikationen wenigstens eine Verbindung der allgemeinen Formel I mit den eingangs genannten Bedeutungen. Sofern die Verbindungen der Formel I ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereömere und Tautomere eingesetzt werden.

Ebenfalls brauchbar sind physiologisch verträgliche Salze der Verbindungen der Formel I, vor allem Säureadditionssalze mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Begriffe wie Alkyl, Alkoxy, etc. umfassen geradkettige oder verzweigte Kohlenwasserstoffgruppen, wie CH₃, C₂H₅, n-Propyl, CH(CH₃)₂, n-Butyl, CH(CH₃)C₂H₅, 2-Methylpropyl, C(CH₃)₃, n-Pentyl oder n-Hexyl, insbesondere CH₃, C₂H₅, CH(CH₃)₂ oder C(CH₃)₃, vorzugsweise mit - soweit nichts anderes angegeben ist - 1 bis 8, insbesondere 1 bis 6 und besonders bevorzugt 1 bis 4 Kohlenstoffatomen.

Zu substituiertem "Alkyl, Alkoxy, etc." gehören insbesondere:

Halogenalkyl, d.h. Alkyl, das partiell oder vollständig, insbesondere 1-, 2-, 3- oder 4-fach durch gleiche oder unterschiedliche Halogenatome, vorzugsweise in α- oder ω-Position, substituiert ist, also z.B. CH₂F, CHF₂, CF₃, CH₂Cl, CF₂Cl, 2-Fluorethyl, 2-Chlorethyl oder 2,2,2-Trifluorethyl, wobei CF₃, CHF₂, CF₃Cl und CH₂F besonders bevorzugt sind;

Halogenalkoxy, d.h. Alkoxy, das partiell oder vollständig, insbesondere 1-, 2-, 3- oder 4-fach durch gleiche oder unterschiedliche Halogenatome, vorzugsweise in α- oder ω-Position, substituiert ist, also z.B. die den vorstehend aufgezählten Halogenalkylresten entsprechenden Halogenalkoxyreste;

Alkoxyalkyl, d.h. durch Alkoxy substituiertes Alkyl, also z.B. CH₂OCH₃ oder 2-Methoxyethyl;

Hydroxyalkyl, d.h. Alkyl, das durch Hydroxy vorzugsweise einfach substituiert ist, z.B. Hydroxymethyl oder 2-Hydroxyethyl;

Phenylalkyl, d.h. Alkyl, das durch Phenyl vorzugsweise einfach substituiert ist, z.B. Benzyl oder Phenylethyl.

Der Begriff "Cycloalkyl" umfasst mono- oder bicyclische, gesättigte Kohlenwasserstoffgruppen, wie Cyclopropyl, Cyclobutyl, Cyclopentyl, Cyclohexyl, etc., vorzugsweise mit - soweit nichts anderes angegeben ist - 3 bis 9, insbesondere 3 bis 6 und besonders bevorzugt 5 oder 6 Kohlenstoffatomen.

Der Begriff "Alkenyl" umfasst geradkettige oder verzweigte, ungesättigte Kohlenwasserstoffgruppen, die vorzugsweise eine Doppelbindung aufweisen, wie Ethenyl, Prop-2-en-1-yl, etc., vorzugsweise mit - soweit nichts anderes angegeben ist - 2 bis 8, insbesondere 2 bis 6 und besonders bevorzugt 2 bis 4 Kohlenstoffatomen.

Der Begriff "Alkinyl" umfasst geradkettige oder verzweigte, ungesättigte Alkylgruppen, die vorzugsweise eine Dreifachbindung aufweisen, wie Ethinyl, Prop-2-in-1-yl, etc., vorzugsweise mit - soweit nichts anderes angegeben ist - 2 bis 8, insbesondere 2 bis 6 und besonders bevorzugt 2 bis 4 Kohlenstoffatomen.

Alkanoyl meint CO-Alkyl, z.B. Acetyl.

Der Begriff "Alkylen" umfasst geradkettige oder verzweigte Reste, wie Methylen, Eth-1,1-ylen, Eth-1,2-ylen, Prop-1,1-ylen, Prop-1,2-ylen, Prop-1,3-ylen, Prop-2,2-ylen, But-1,1-ylen, But-1,2-ylen, But-1,3-ylen, But-1,4-ylen, But-2,2-ylen, 2-Methyl-prop-1,3-ylen, Pent-1,1-ylen, Pent-1,2-ylen, Pent-1,3-ylen, Pent-1,4-ylen, Pent-1,5-ylen, Pent-2,2-ylen, Pent-2,3-ylen, Pent-2,4-ylen, Pent-3,3-ylen, 1-Methylbut-1,4-ylen, 2-Methyl-but-1,4-ylen, etc., vorzugsweise mit - soweit nichts anderes angegeben ist - 1 bis 18, insbesondere 3 bis 10 und besonders bevorzugt 3 bis 8 Kohlenstoffatomen. In Zusammenhang mit der Gruppe D können diese Reste eine oder mehrere Reste Z umfassen, so daß sich Alkylenreste ergeben können, deren Kohlenstoffkette durch einen oder mehrere Reste Z unterbrochen ist oder in denen gesättigte Bindungen durch ungesättigte Bindungen (Alkenylen; Alkinylen) ersetzt sind. So können sich geradkettige oder verzweigte ungesättigte Reste ergeben, deren Anzahl und Anordnung der Kohlenstoffatome derjenigen der zuvor genannten Alkylenreste entspricht, wobei jeodch eine oder mehrere Einfachbindungen durch entsprechende ungesättigte Doppel- bzw. Dreifachbindungen ersetzt sind.

Der Begriff "Halogen" umfasst ein Fluor-, Chlor-, Brom- oder Iodatom und insbesondere ein Fluor- oder Chloratom.

Der Begriff "heterocyclischer Rest" umfasst insbesondere 5- und 6-gliedrige heterocyclische Ringe, die aromatisch oder nicht-aromatisch, mono- oder bicyclisch, und/oder benzoannelliert sein können, mit vorzugsweise - soweit nichts anderes angegeben ist - 1, 2, 3 oder 4, gleichen oder verschiedenen, unter O, S und N ausgewählten Heteroatomen. Hierzu gehören vor allem Pyridinyl, Pyrimidinyl, Pyrazinyl, Imidazolyl, Indolyl, Benzofuranyl, Benzothienyl, Pyrrolyl, Furanyl, Pyrazolyl, Thienyl, Oxazolyl, Thiazolyl, Isoxazolyl, Tetrazolyl, Triazinyl, Thiadiazolyl und Triazolyl.

Die vorstehend beschriebenen Verbindungen der allgemeinen Formel I können hergestellt werden, indem man
a) eine Verbindung der allgemeinen Formel (II)

   L - D - Y¹

   worin Y¹ für eine übliche Abgangsgruppe steht, wie beispielsweise Halogen, Alkansulfonyloxy, Arylsulfonyloxy etc., und Z die oben genannten Bedeutungen besitzt,
   mit einer Verbindung der allgemeinen Formel (III)

   H - B - G

   umsetzt; oder
b) eine Verbindung der allgemeinen Formel (IV)

   L - D1 - Z¹H (IV)

   worin Z¹ für O, NR¹ oder S und D1 für C₁-C₁₀-Alkylen oder eine Bindung steht, mit einer Verbindung der allgemeinen Formel V

   Y¹ - D2 - B - G (V)

   wobei Y¹ die oben angegebene Bedeutung besitzt und D2 für C₂-C₁₀-Alkylen steht, wobei D1 und D2 zusammen 1 bis 18 C-Atome aufweisen,
   umsetzt; oder
c) eine Verbindung der allgemeinen Formel (VI)

   L - Y¹ (VI)

   worin Y¹ die oben angegebene Bedeutung besitzt, mit einer Verbindung der allgemeinen Formel VII

   H - Z¹ - D - B - G (VII)

   worin Z¹ die oben angegebenen Bedeutungen besitzt, umsetzt; oder
d) eine Verbindung der Formel (VIII)

   NC - D - B - G (VIII)

   in eine Verbindung des Typs (IX) 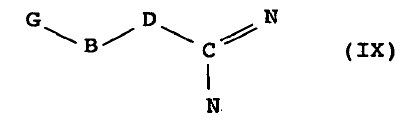 überführt,
   und diese mit einer Dicarbonyl-Verbindung in bekannter Weise umsetzt; oder
e) eine Verbindung der allgemeinen Formel (X) 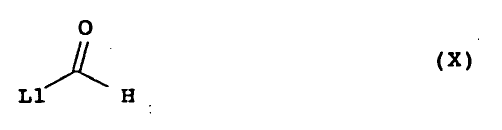 mit literaturbekannten Reagenzien, wie z.B. 1,3-Propandithiol, KCN/Wasser, TMSCN (Trimethylsilylcyanid) oder KCN/Morpholin, wie z.B. beschrieben in
   Albright, Tetrahedron, 1983, 39, 3207 oder D. Seebach, Synthesis 1969, 17 und 1979, 19 oder H. Stetter, Angew. Chem. Int. Ed. 1976, 15, 639 oder van Niel et al., Tetrahedron 1989, 45, 7643 Martin et al., Synthesis 1979, 633,
   zu den Produkten (Xa) (exemplarisch mit 1,3-Propandithiol) 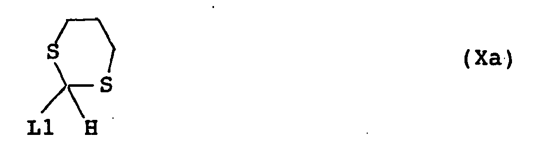 umpolt und anschließend mit Verbindungen der allgemeinen Formel (XI)

   Y¹ - D3 - B - G (XI)

   wobei Y¹ die oben angegebene Bedeutung besitzt und D3 für C₃-C₉-Alkylen steht, das eine Gruppe Z enthalten kann, kettenverlängert, wobei man nach Entschützen oder Reduktion Verbindungen der Formel (Ia)

   T - Z³ - D2 - B - G (Ia)

   worin Z³ für CO oder eine Methylengruppe steht und Z³ und D2 zusammen 4 bis 10 C-Atome aufweisen, erhält; oder
g) eine Verbindung der Formel (X) mit einer Verbindung der allgemeinen Formel (XII)

   Y³- D - B - G (XII)
worin Y³ für ein Phosphoran oder einen Phosphonsäureester steht, analog zu üblichen Methoden, wie zum Beispiel beschrieben in Houben Weyl "Handbuch der Organischen Chemie" 4. Auflage, Thieme Verlag Stuttgart, Band V/1b S. 383 ff oder Bd V/1c S. 575 ff, umsetzt.

Ein Verfahren zur Herstellung einer Verbindung der Formel I, die die Gruppe COO oder CONR⁷ umfaßt, kann darin bestehen, daß man eine Verbindung der allgemeinen Formel (XIII) 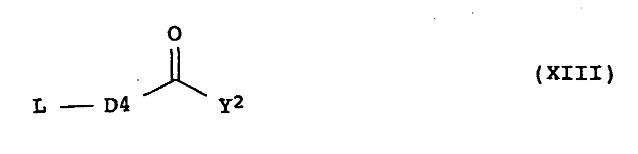 worin Y² für OH, OC₁-C₄-Alkyl, Cl oder zusammen mit CO für eine aktivierte Estergruppe und D4 für C₀-C₉-Alkylen, das eine Gruppe Z enthalten kann, steht, mit einer Verbindung der Formel (XIV)

Z² - D - B - G (XIV)

worin Z² für OH oder NR⁷ steht, umsetzt, wobei L, D, B und G die oben angegebenen Bedeutungen besitzen.

Die Verbindungen der Formel III sind Ausgangsverbindungen zur Herstellung von Verbindungen der Formeln V, VII, VIII.

Verbindungen der Formel III werden hergestellt nach Standardmethoden, wie z.B. beschrieben in J.A. Kiristy et al., J. Med. Chem. 1978, 21, 1303 oder C.B. Pollard, J. Am. Chem. Soc. 1934, 56, 2199,
oder indem man
a) eine Verbindung der allgemeinen Formel (XV)

   HB3 (XV)

   worin B3 für 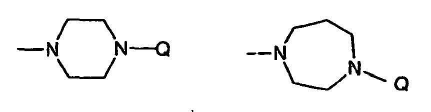 oder 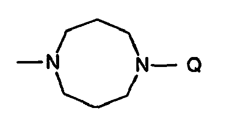 und Q für H oder eine übliche Aminoschutzgruppe z.B. Butyloxycarbonyl, Benzyl, oder Methyl, steht, mit einer Verbindung der allgemeinen Formel (XVI)

   Y⁴ - Gx (XV)

   worin Y⁴ für B(OH)₂, -SnBu₃, Trifluormethansulfonyloxy steht oder die für Y¹ angegebenen Bedeutungen besitzt in bekannter Weise umsetzt; oder
b) eine Verbindung der allgemeinen Formel (XVII)

   Q - B4 (XVII)

   worin B4 für 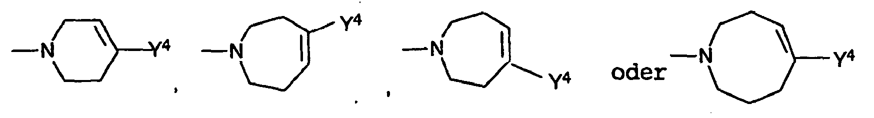 steht und Y⁴ und Q die oben angegebene Bedeutung besitzen mit einer Verbindung der allgemeinen Formel (XVIII)

   Y⁵ - G (XVIII)

   worin Y⁵ für Borderivate, wie z.B. B(OH)₂ oder eine metallhaltige Abgangsgruppe, z.B. SnR³ (R = Butyl oder Phenyl) oder Zinkhalogenid steht, wenn Y⁴ für Halogen oder Trifluormethylsulfonyloxy steht; oder worin Y⁵ für Halogen oder Trifluormethylsulfonyloxy steht, wenn Y⁴ für Borderivate, wie z.B. B(OH)₂ oder eine metallhaltige Abgangsgruppe, z.B. SnR³ (R = Butyl oder Phenyl) oder Zinkhalogenid, steht, nach bekannten Verfahren umsetzt, wie z.B. beschrieben in
   S. Buchwald et al., Angew. Chem. 1995, 107, 1456 oder J.F. Hartweg et al., Tetrahedron Lett. 1995, 36, 3604 bzw. J.K. Stille et al., Angew. Chem. 1986, 98, 504 oder Pereyre M. et al., "Tin in Organic Synthesis", Butterworth 1987; oder
c) eine Verbindung der allgemeinen Formel (XIX) 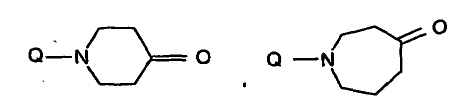 oder 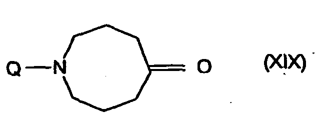 mit einer Verbindung M - G,
worin M für ein Metall wie z.B. Li, MgY⁶ und Y⁶ für Brom, Chlor oder Jod steht, umsetzt.

M-G kann nach literaturbekannten Methoden erhalten werden.

Verbindungen des Typs B sind entweder bekannt oder sie können analog zu bekannten Verfahren hergestellt werden, wie z.B.

1,4- und 1,5-Diazacycloalkane: L. Börjeson et al. Acta Chem. Scand. 1991, 45, 621 Majahrzah et al Acta Pol. Pharm. 1975, 32, 145

1,4-Diazacyclooct-6-ene:
W. Schroth et al. Z. Chem. 1969, 9, 143

1-Azacyclooctanone:
N.J. Leonard et al. J. Org. Chem. 1964, 34, 1066

1-Azacycloheptanone:
A. Yokoo et al. Bull Chem. Soc. Jpn. 1956, 29, 631

Verbindungen des Typs L sowie G sind entweder bekannt oder können nach bekannten Verfahren hergestellt werden wie z.B. beschrieben in A.R. Katritzky, C.W. Rees (ed.) "Comprehensive Heterocyclic Chemistry", Pergamon Press, oder "The Chemistry of Heterocyclic Compounds", J. Wiley & Sons Inc. NY und der dort zitierten Literatur oder der vorstehend zitierten Patentliteratur.

Die Verbindungen des Typs der Formel (IV) sind entweder bekannt oder können nach bekannten Verfahren hergestellt werden, wie z.B. beschrieben in A.R. Katritzky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. Pharm. Bull. 1975, 23, 955 oder Vosilevskii et al. Izv. Akad. Nauk. SSSR Ser. Khim. 1975, 23, 955.

Weitere Synthesemöglichkeiten ergeben sich dem Fachmann aus den in WO 96/02519, WO 96/02520, WO 96/02249, WO 96/02246, WO 97/25324, WO 99/02503 und WO 98/05178 geschilderten Verfahren und insbesondere aus den dort angegebenen erläuternden Herstellungsbeispielen.

Von den vorstehend genannten Verbindungen sind erfindungsgemäß insbesondere diejenigen von Vorteil, die eine hohe Affinität zu Dopamin-D₃-Rezeptoren besitzen. In diesem Sinne besonders bevorzugt sind Verbindungen, die in vitro Kᵢ-Werte von weniger als 1 µM und vor allem von weniger als 200 nM besitzen. Geeignete Testverfahren zur Auswahl dieser Verbindungen sind dem Fachmann bekannt. Beispielsweise können Bindungsaffinitäten zu D₃-Rezeptoren in Rezeptorbindungsstudien über die Verdrängung von [¹²⁵I]-Iodosulpirid bestimmt werden.

Insbesondere von Vorteil sind diejenigen der zuvor beschriebenen Verbindungen, die selektiv an Dopamin-D₃-Rezeptoren binden. Von Bedeutung sind in diesem Zusammenhang vor allem Selektivitäten gegenüber D₁-Rezeptoren, D₄-Rezeptoren, α1- und/oder α2-adrenergen-Rezeptoren, serotinergen Rezeptoren, vor allem 5HT1A und 5HT7, muskarinergen Rezeptoren, histaminischen Rezeptoren, Opiatrezeptoren und insbesondere gegenüber Dopamin-D₂-Rezeptoren.

Auch für diese Rezeptoren sind dem Fachmann einschlägige Testverfahren zur Bestimmung von Bindungsaffinitäten bekannt. Rezeptorbindungsstudien an D₁-, D₂- und D₄-Rezeptoren können beispielsweise über die Verdrängung von [³H]SCH23390, [¹²⁵I]Iodosulpirid bzw. [³H]Spiperon vorgenommen werden.

Erfindungsgemäß von besonderer Bedeutung ist die Selektivität Kᵢ(D₂)/Kᵢ(D₃), die vorzugsweise wenigstens 10, besser noch wenigstens 50 und besonders vorteilhaft wenigstens 100 beträgt.

Im Hinblick auf die Effektorfunktion verwendet man gemäß einer besonderen Ausführungsform der vorliegenden Erfindung diejenigen der vorstehend genannten Verbindungen, die partielle Dopamin-D₃-Rezeptor-Agonisten darstellen.

Auch zur Ermittlung von Effektorfunktionen stehen dem Fachmann hinlänglich bekannte Testverfahren zur Verfügung. Beispielsweise führen D₃-Rezeptor-Agonisten in D₃-exprimierenden Zellen zu einer Abnahme intrazellulärer cAMP-Spiegel, die direkt über die an sich bekannte Bestimmung von cAMP oder indirekt beispielsweise über cAMP-abhängige Reporter bestimmt werden können. Ein weiteres Beispiel ist die Erhöhung der Affinität der α-Untereinheit von G-Proteinen für GTP, die über die Stimulierung der Bindung von ³⁵S-GTP an G-Proteine gemessen werden kann. Die Stimulierung des ³H-Thymidin-Einbaus in D₃-Rezeptor exprimierenden Neuroblastomazellen durch D₃-Rezeptor-Agonisten und die Veränderung intrazellulärer pH-Werte durch Veränderungen der Säureexkretion sind weitere Möglichkeiten, Effektorfunktionen erfindungsgemäß zu verwendender Verbindungen zu bewerten.

Geeignete Modelle im Bereich der Suchterkrankungen basieren auf suchttypischen Verhaltensweisen von Tieren, denen psychotrope Substanzen verabreicht werden. Beispielsweise werden Tiere darauf trainiert, bei Behandlung mit Wirkstoff eine Taste und bei Behandlung mit Placebo eine andere Taste zu drücken. Die Testsubstanz wird auf ihre Fähigkeit untersucht, ein derartiges Verhalten zu induzieren, die durch eine weitere psychotrope Substanz induzierte Antwort zu supprimieren oder eine weitere psychotrope Substanz zu ersetzen. In ähnlicher Weise können die Tiere dahingehend konditioniert werden, daß sie einen bestimmten Ort bevorzugen. Ein weiteres Beispiel basiert auf der Fähigkeit eines Wirkstoffes, ein Tier dazu zu bringen, sich diesen Wirkstoff selbst zu verabreichen, gewöhnlicherweise durch Aktivierung einer Pumpe, die an einen Katheter angeschlossen ist. Auch die Fähigkeit eines Wirkstoffs, im Anschluß an eine chronische Verabreichung bei Entzug physische Symptome hervorzurufen, stellt eine Möglichkeit dar, das psychotrope Potential bzw. die Fähigkeit, der psychotropen Wirkung eines bestimmten Wirkstoffes entgegenzuwirken, zu beurteilen.

Ganz besonders vorteilhaft sind diejenigen Verbindungen, die sowohl die vorstehend beschriebenen vorteilhaften Bindungseigenschaften an Dopamin-D₃-Rezeptoren aufweisen, als auch eine oder mehrere der beispielhaft beschriebenen Effektorfunktionen ausüben. Vorzugsweise führen solche Verbindungen in D₂-exprimierenden Zellen nicht oder erst in wesentlich höherer Konzentration zu den vorstehend beschriebenen Effekten.

Von Vorteil sind Verbindungen, die selbst keine psychotrope Wirkung besitzen. Dies kann im Test auch an Ratten beobachtet werden, die nach Verabreichung erfindungsgemäß brauchbarer Verbindungen die Selbstverabreichung von psychotropen Substanzen, beispielsweise Kokain, drosseln.

Die vorstehend beschriebenen und weitere in ähnlicher Weise geeignete Testsysteme können die Grundlage bilden für in vitro-Screening-Verfahren, vorzugsweise zum primären Screening, mit denen man aus den beschriebenen Verbindungen diejenigen auslesen kann, die im Hinblick auf die erfindungsgemäße Anwendung besondere Vorteile bieten. Dies ist automatisierbar. Screening-Roboter dienen der effizienten Auswertung der vorzugsweise auf Mikrotiterplatten angeordneten Einzelassays.

Eine besonders effektive Technologie zur Durchführung derartiger Verfahren ist der im Bereich des Wirkstoffscreenings bekannte Scintillation Proximity Assay, kurz SPA genannt. Kits und Komponenten zur Durchführung dieses Assays können kommerziell bezogen werden, beispielweise bei Amersham Pharmacia Biotech.

Eine weitere besonders effektive Technologie zur Durchführung derartiger Verfahren ist die im Bereich des Wirkstoffscreenings bekannte FlashPlate-Technologie. Kits und Komponenten zur Durchführung dieses Assays können kommerziell bezogen werden, beispielweise bei NEN Life Science Products. Dieses Prinzip basiert ebenfalls auf Mikrotiterplatten (96er oder 384er), die mit Scintillationssubstanz beschichtet sind.

Weitere, vor allem zum sekundären Screening geeignete Testverfahren beruhen auf in-vitro und in-vivo Modellen für erfindungsgemäß zu behandelnde Indikationen.

Die erfindungsgemäße Verwendung der beschriebenen Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabfolgung gegebenenfalls zusammen oder im Wechsel mit anderen Wirkstoffen oder wirkstoffhaltigen Präparaten, so daß einem zu behandelnden Individuum eine Tagesdosis von vorzugsweise etwa 1 bis 1000 mg/kg Körpergewicht bei oraler Gabe bzw. von etwa 0,1 bis 100 mg/kg Körpergewicht bei parenteraler Gabe zugeführt wird.

Die Erfindung betrifft auch die Herstellung pharmazeutischer Mittel zur Behandlung eines Individuums, vorzugsweise eines Säugers, insbesondere eines Menschen, Nutz- oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Inhibitoren verwendet werden. Ferner können auch Liposomen oder Mikrosphären zur Anwendung kommen.

Bei der Herstellung der Zusammensetzungen werden erfindungsgemäße Inhibitoren gewöhnlich mit einem Exzipienten vermischt oder verdünnt. Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme-oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die vorliegende Erfindung wird anhand der nachfolgenden Beispiele näher erläutert.

### Beispiel 1

5-({3-[4-(3,5-Dichlorphenyl)-1-piperazinyl]propyl}sulfanyl)-1,3,4-thiadiazol-2-amin;

### Beispiel 2

5-[(2-Methyl-3-(4-[3-(trifluormethyl)phenyl]-1-piperazinyl)propyl)sulfanyl]-1,3,4-thiadiazol-2-amin;

### Beispiel 3

2-[{3-(4-[3-(Trifluormethyl)phenyl]-1-piperazinyl}propyl)sulfanyl]-4-pyrimidinol;

### Beispiel 4

5-(6-(4-[3-(Trifluormethyl)phenyl]-1-piperazinyl)hexyl)-1,3,4-thiadiazol-2-amin;

### Beispiel 5

1-{(2E)-4-[(4-Hydroxy-2-pyrimidinyl)sulfanyl]-2-butenyl}-4-[3-(trifluormethyl)phenyl]piperazindiium-dichlorid;

### Beispiel 6

4-Methyl-5-[(3-(4-[3-(trifluormethyl)phenyl]-1-piperazinyl)propyl)sulfanyl]-4H-1,2,4-triazol-3-amin;

### Beispiel 7

2-([3-(4-[3-(Trifluormethyl)phenyl]-3,6-dihydro-1(2H)-pyridinyl)propyl]sulfanyl)-4-pyrimidinol;

### Beispiel 8

2-({3-[4-(3-Isopropylphenyl)-1-piperazinyl]propyl}sulfanyl)-4-pyrimidinol;

### Beispiel 9

5-[(3-{4-[6-(Trifluormethyl)-2-pyridinyl]-1-piperazinyl}propyl)sulfanyl}-1,3,4-thiadiazol-2-amin;

### Beispiel 10

5-[((2E)-4-(4-[6-(Trifluormethyl)2-pyridinyl]-1-piperazinyl}-2-butenyl)sulfanyl]-1,3,4-thiadiazol-2-amin;

### Beispiel 11

5-({3-[4-(3,5-Ditert-butylphenyl)1-piperazinyl]propyl}sulfanyl)-4-methyl-4H-1,2,4-triazol-3-amin;

### Beispiel 12

5-({7-[4-(3-Isopropylphenyl)-1-piperazinyl]heptyl}sulfanyl)-1,3,4-thiadiazol-2-amin;

### Beispiel 13

4-Ethyl-5-[(3-(4-[3-(trifluormethyl)phenyl]-1-piperazinyl)propyl)sulfanyl]-4H-1,2,4-triazol-3-amin;

### Beispiel 14

2-[(3-{4-[2-tert-Butyl-6-(trifluormethyl)-4-pyrimidinyl]-1-piperazinyl}propyl)sulfanyl]-4-pyrimidinol, insbesondere dessen Fumarsäuresalz;

### Beispiel 15

3-(4-{8-[(5-Amino-1,3,4-thiadiazol-2-yl)sulfanyl]octyl)-1-piperazinyl)benzonitril;

### Beispiel 16

5-[(3-{4-[6-Methyl-2-(1H-pyrrol-1-yl)-4-pyrimidinyl]-1-piperazinyl}propyl)sulfanyl]-1,3,4-thiadiazol-2-amin;

### Beispiel 17

4-Methyl-5-[(3-{4-[6-Methyl-2-(1H-pyrrol-1-yl)-4-pyrimidinyl]-1-piperazinyl}propyl)sulfanyl]-4H-1,2,4-triazol-3-amin;

### Beispiel 18

5-[{(2E)-4-(4-[2-tert-Butyl-6-(trifluormethyl)-4-pyrimidinyl]-1-piperazinyl)-2-methyl-2-butenyl}sulfanyl]-4-methyl-4H-1,2,4-triazol-3-amin;

### Beispiel 19

1-[3-(Difluormethyl)phenyl)-4-(3-([4-methyl-5-(methylammonio)-4H-1,2,4-triazol-3-yl]sulfanyl)propyl)piperazin-4-ium-di-chlorid;

### Beispiel 20

5-({3-[4-(2,6-Ditert-butyl-4-pyrimidinyl)-1-piperazinyl]propyl}sulfanyl)-1,3,4-thiadiazol-2-amin.

Die in den Beispielen 1 bis 20 aufgeführten Verbindungen sind selektive Dopamin-D₃-Liganden, deren Affinität zu D₂-Rezeptoren bzw. D₃-Rezeptoren gemäß den in den Referenzbeispielen angegebenen Methoden zu einem Verhältnis Kᵢ(D₂)/Kᵢ(D₃) von mehr als 10 bestimmt wurde.

### Referenzbeispiele

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10% fötalem Kälberserum (GIBCO Nr. 041-32400 N); 100 E/ml Penicillin und 0,2% Streptomycin (GIBCO BRL, Gaithersburg, MD, USA) vermehrt. Nach 48 h wurden die Zellen mit PBS gewaschen und mit 0,05% trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5mM Tris-HCl, pH 7,4 mit 10% Glycerin) gewaschen und danach in einer Konzentration von 10⁷-Zellen/ml Lysispuffer 30 min bei 4°C inkubiert. Die Zellen wurden bei 200 g 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10 µM Quinolinol, 0,1% Ascorbinsäure und 0,1% BSA) in einer Konzentration von ca. 10⁶ Zellen/250 µl Testansatz suspendiert und bei 30°C mit 0,1 nM ¹²⁵jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Ki-Werte erfolgte über nichtlineare Regressionsanalyse mit dem Programm LIGAND.

### 2) D₂-Bindungstest

### Zellkultur

HEK-293 Zellen mit stabil exprimierten humanen Dopamin-D2A-Rezeptoren wurden in RPMI 1640 mit Glutamax I™ und 25 mM HE-PES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37°C gehalten.

Die Zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4 °C mit Lysispuffer (5 mM Tris-HCl, 10% Glycerol, pH 7,4) behandelt. Nach Zentrifugation bei 250 g für 10 Minuten wurde der Rückstand bei -20°C bis zum Gebrauch aufbewahrt.

### Rezeptorbindungstests

Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl, 5 mM KCl, 2 mM MgCl₂ und 2 mM CaCl₂, pH 7,4 mit HCl) und 0,1 nM ¹²⁵I-Spiperon (totale Bindung) oder zusätzlich 1 µM Haloperidol (unspezifische Bindung) oder Prüfsubstanz.

Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

Die Auswertung erfolgte wie unter a).

Die Bestimmung der Kᵢ-Werte erfolgte über nichtlineare Regressionsanalyse mit dem Programm LIGAND oder durch Umrechnung der IC₅₀-Werte mit Hilfe der Formel von Cheng und Prusoff.

Die erfindungsgemäßen Verbindungen zeigen in diesen Tests sehr gute Affinitäten am D₃-Rezeptor (< 1 µmolar, insbesondere < 100 nmolar) und hohe Selektivitäten gegenüber dem D₂-Rezeptor.

## Patentansprüche

1. Verwendung von Verbindungen, die ausgewählt sind unter
2-[(3-{4-[2-tert-Butyl-6-(trifluormethyl)-4-pyrimidinyl]-1-piperazinyl}propyl)sulfanyl]-4-pyrimidinol,
5-({3-[4-(3,5-Dichlorphenyl)-1-piperazinyl]propyl}sulfanyl)-1,3,4-thiadiazol-2-amin,
5-[(2-Methyl-3-(4-[3-(trifluormethyl)phenyl]-1-piperazinyl)propyl)sulfanyl]-1,3,4-thiadiazol-2-amin,
2-[{3-(4-[3-(Trifluormethyl)phenyl]-1-piperazinyl}propyl)sulfanyl]-4-pyrimidinol,
5-(6-(4-[3-(Trifluormethyl)phenyl]-1-piperazinyl)hexyl)-1,3,4-thiadiazol-2-amin,
1-{(2E)-4-[(4-Hydroxy-2-pyrimidinyl)sulfanyl]-2-butenyl}-4-[3-(trifluormethyl)phenyl]piperazindiium-dichlorid,
4-Methyl-5-[(3-(4-[3-(trifluormethyl)phenyl]-1-piperazinyl)propyl)sulfanyl]-4H-1,2,4-triazol-3-amin,
2-([3-(4-[3-(Trifluormethyl)phenyl]-3,6-dihydro-1(2H)-pyridinyl)propyl]sulfanyl)-4-pyrimidinol,
2-({3-[4-(3-Isopropylphenyl)-1-piperazinyl]propyl}sulfanyl)-4-pyrimidinol,
5-[(3-{4-[6-(Trifluormethyl)-2-pyridinyl]-1-piperazinyl}propyl)sulfanyl}-1,3,4-thiadiazal-2-amin,
5-[((2E)-4-(4-[6-(Trifluormethyl)2-pyridinyl]-1-piperazi-nyl}-2-butenyl)sulfanyl]-1,3,4-thiadiazol-2-amin,
5-({3-[4-(3,5-Ditert-butylphenyl)l-piperazinyl]propyl}sulfanyl)-4-methyl-4H-1,2,4-triazol-3-amin,
5-({7-[4-(3-Isopropylphenyl)-1-piperazinyl]heptyl}sulfanyl)-1,3,4-thiadiazol-2-amin,
4-Ethyl-5-[(3-(4-[3-(trifluormethyl)phenyl]-1-piperazinyl)propyl)sulfanyl]-4H-1,2,4-triazol-3-amin,
3-(4-{8-[(5-Amino-1,3,4-thiadiazol-2-yl)sulfanyl]octyl)-1-piperazinyl)benzonitril,
5-[(3-{4-[6-Methyl-2-(1H-pyrrol-1-yl)-4-pyrimidinyl]-1-piperazinyl}propyl)sulfanyl]-1,3,4-thiadiazol-2-amin,
4-Methyl-5-[(3-{4-[6-Methyl-2-(1H-pyrrol-1-yl)-4-pyrimidinyl]-1-piperazinyl)propyl)sulfanyl]-4H-1,2,4-triazol-3-amin,
5-[{(2E)-4-(4-[2-tert-Butyl-6-(trifluormethyl)-4-pyrimidinyl]-1-piperazinyl)-2-methyl-2-butenyl}sulfanyl]-4-methyl-4H-1,2,4-triazol-3-amin,
1-[3-(Difluormethyl)phenyl)-4-(3-([4-methyl-5-(methylammonio)-4H-1,2p4-triazo1-3-yl]sulfanyl)propyl)piperazin-4-ium-di-chlorid und
5-({3-[4-(2,6-Ditert-butyl-4-pyrimidinyl)-1-piperazinyl]propyl}sulfanyl)-1,3,4-thiadiazol-2-amin,
oder deren Salze mit physiologisch verträglichen Säuren,
zur Herstellung eines Medikaments zur Behandlung von Sucht.

2. Verwendung nach Anspruch 1 zur Behandlung von Störungen, die durch psychotrope Substanzen, insbesondere durch Opioide oder Kokain, vermittelt werden.

3. Verwendung nach einem der Ansprüche 1 oder 2, von
2-[(3-{4-[2-tert-Butyl-6-(trifluormethyl)-4-pyrimidinyl]-1-piperazinyl}propyl)sulfanyl]-4-pyrimidinol oder dessen Salze mit physiologisch verträglichen Säuren.

4. Verwendung nach Anspruch 3, wobei es sich bei den Salzen mit physiologisch verträglichen Säuren um das Fumarsäuresalz handelt.

## Claims

1. The use of compounds selected from
2-[(3-{4-[2-tert-butyl-6-(trifluoromethyl)-4-pyrimidinyl]-1-piperazinyl}propyl)sulfanyl]-4-pyrimidinole,
5-({3-[4-(3,5-dichlorophenyl)-1-piperazinyl]-propyl}sulfanyl)-1,3,4-thiadiazole-2-amine,
5-[(2-methyl-3-(4-[3-(trifluoromethyl)phenyl]-1-piperazinyl)propyl)sulfanyl]-1,3,4-thiadiazole-2-amine,
2-[{3-(4-[3-(trifluoromethyl)phenyl]-1-piperazinyl}propyl)sulfanyl]-4-pyrimidinole,
5-(6-(4-[3-(trifluoromethyl)phenyl]-1-piperazinyl)hexyl)-1,3,4-thiadiazole-2-amine,
1-{(2E)-4-[(4-hydroxy-2-pyrimidinyl)sulfanyl]-2-butenyl}-4-[3-(trifluoromethyl)phenyl]-piperazinediium dichloride,
4-methyl-5-[(3-(4-[3-(trifluoromethyl)phenyl]-1-piperazinyl)propyl)sulfanyl]-4H-1,2,4-triazole-3-amine,
2-([3-(4-[3-(trifluoromethyl)phenyl]-3,6-dihydro-1(2H)-pyridinyl)propyl]sulfanyl)-4-pyrimidinole,
2-({3-[4-(3-isopropylphenyl)-1-piperazinyl]-propyl}sulfanyl)-4-pyrimidinole,
5-[(3-{4-[6-(trifluoromethyl)-2-pyridinyl]-1-piperazinyl}propyl)sulfanyl}-1,3,4-thiadiazole-2-amine,
5-[((2E)-4-(4-[6-(trifluoromethyl)-2-pyridinyl]-1-piperazinyl}-2-butenyl)sulfanyl]-1,3,4-thiadiazole-2-amine,
5-({3-[4-(3,5-di-tert-butylphenyl)-1-piperazinyl]-propyl}sulfanyl)-4-methyl-4H-1,2,4-triazole-3-amine,
5-({7-[4-(3-isopropylphenyl)-1-piperazinyl]-heptyl}sulfanyl)-1,3,4-thiadiazole-2-amine,
4-Ethyl-5-[(3-(4-[3-(trifluoromethyl)phenyl]-1-piperazinyl)propyl)sulfanyl]-4H-1,2,4-triazole-3-amine,
3-(4-{8-[(5-amino-1,3,4-thiadiazol-2-yl)sulfanyl]-octyl)-1-piperazinyl)benzonitrile,
5-[(3-f4-[6-methyl-2-(1H-pyrrol-1-yl)-4-pyrimidinyl]-1-piperazinyl}propyl)sulfanyl]-1,3,4-thiadiazole-2-amine,
4-methyl-5-[(3-(4-[6-methyl-2-(1H-pyrrol-1-yl)-4-pyrimidinyl]-1-piperazinyl}propyl)sulfanyl]-4H-1,2,4-triazole-3-amine,
5-[{(2E)-4-(4-[2-tert-butyl-6-(trifluoromethyl)-4-pyrimidinyl]-1-piperazinyl)-2-methyl-2-butenyl}-sulfanyl]-4-methyl-4H-1,2,4-triazole-3-amine,
1-[3-(difluoromethyl)phenyl)-4-(3-([4-methyl-5-(methylammonio)-4H-1,2,4-triazole-3-yl)sulfanyl)-propyl)piperazin-4-ium dichloride and
5-({3-[4-(2,6-di-tert-butyl-4-pyrimidinyl)-1-piperazinyl]propyl}sulfanyl)-1,3,4-thiadiazole-2-amine,
or their salts with physiologically tolerable acids,
for the production of a medicament for treating addiction.

2. The use according to claim 1 for the treatment of disorders which are mediated by psychotropic substances, in particular by opioids or cocaine.

3. The use according to either of claims 1 and 2 of
2-[(3-{4-[2-tert-butyl-6-(trifluoromethyl)-4-pyrimidinyl]-1-piperazinyl}propyl)sulfanyl]-4-pyrimidinole or its salts with physiologically tolerable acids.

4. The use according to claim 3, wherein the salts with physiologically tolerable acids are the fumaric acid salt.

## Revendications

1. Utilisation de composés, qui sont choisis parmi :
le 2-[(3-{4-[2-tert-butyl-6-(trifluorométhyl)-4-pyrimidinyl]-1-pipérazinyl}propyl)sulfanyl]-4-pyrimidinol,
la 5-({3-[4-(3,5-dichlorophényl)-1-pipérazinyl]propyl}sulfanyl)-1,3,4-thiadiazol-2-amine,
la 5-[(2-méthyl-3-(4-[3-(trifluorométhyl)phényl]-1-pipérazinyl)-propyl)sulfanyl]-1,3,4-thiadiazol-2-amine,
le 2-[{3-(4-[3-(triflurométhyl)phényl]-1-pipérazinyl)propyl}-sulfanyl]-4-pyrimidinol,
la 5-(6-(4-[3-trifluorométhyl)phényl]-1-pipérazinyl)hexyl)-1,3,4-thiadiazol-2-amine,
le dichlorure de 1-{(2E)-4-[(4-hydroxy-2-pyrimidinyl)sulfanyl]-2-butényl}-4-[3-(trifluorométhyl)phényl]pipérazindiium,
la 4-méthyl-5-[(3-(4-[3-(trifluorométhyl)phényl]-1-pipérazinyl)-propyl)sulfanyl]-4H-1,2,4-triazol-3-amine,
le 2-([3-(4-[3-(trifluorométhyl)phényl]-3,6-dihydro-1(2H)-pyridinyl)propyl]sulfanyl)-4-pyrimidinol,
le 2-({3-[4-[3-isopropylphényl)-1-pipérazinyl]propyl}sulfanyl)-4-pyrimidinol,
la 5-[(3-{4-[6-(trifluorométhyl)-2-pyridinyl]-1-pipérazinyl}-propyl)sulfanyl}-1,3,4-thiadiazol-2-amine,
la 5-[((2E)-4-(4-[6-(trifluorométhyl)-2-pyridinyl]-1-pipérazinyl}-2-butényl)sulfanyl]-1,3,4-thiadiazol-2-amine,
la 5-({3-[4-(3,5-ditert-butylphényl)-1-pipérazinyl]propyl}sulfanyl)-4-méthyl-4H-1,2,4-triazol-3-amine,
la 5-({7-[4-(3-isopropylphényl)-1-pipérazinyl]heptyl}sulfanyl)-1,3,4-thiadiazol-2-amine,
la 4-éthyl-5-[(3-(4-[3-(trifluorométhyl)phényl]-1-pipérazinyl)-propyl)sulfanyl]-4H-1,2,4-triazol-3-amine,
le 3-(4-{8-[(5-amino-1,3,4-thiadiazol-2-yl)sulfanyl]octyl}-1-pipérazinyl)benzonitrile,
la 5-[(3-{4-[6-méthyl-2-(1H-pyrrol-1-yl)-4-pyrimidinyl]-1-pipérazinyl}propyl)sulfanyl]-1,3,4-thiadiazol-2-amine,
la 4-méthyl-5-[(3-{4-[6-méthyl-2-(1H-pyrrol-1-yl)-4-pyrimidinyl]-1-pipérazinyl}propyl)sulfanyl]-4H-1,2,4-triazol-3-amine,
la 5-[{(2E)-4-(4-[2-tert-butyl-6-(trifluorométhyl)-4-pyrimidinyl]-1-pipérazinyl)-2-méthyl-2-butényl}sulfanyl]-4-méthyl-4H-1,2,4-triazol-3-amine,
le dichlorure de 1-[3-(difluorométhyl)phényl)-4-(3-([4-méthyl-5-(méthylammonio)-4H-1,2,4-triazol-3-yl]sulfanyl)propyl)pipérazin-4-ium et
la 5-({3-[4-(2,6-ditert-butyl-4-pyrimidinyl)-1-pipérazinyl]-propyl}sulfanyl)-1,3,4-thiadiazol-2-amine,
ou de sels de ceux-ci avec des acides physiologiquement compatibles,
pour la préparation d'un médicament destiné au traitement de la toxicomanie/dépendance.

2. Utilisation selon la revendication 1 pour le traitement de troubles qui sont occasionnés par des substances psychotropes, en particulier par des opioïdes ou la cocaïne.

3. Utilisation selon l'une quelconque des revendications 1 ou 2 de 2-[(3-{4-(2-tert-butyl-6-(trifluorométhyl)-4-pyrimidinyl]-1-pipérazinyl}-propyl)sulfanyl]-4-pyrimidinol ou de sels de celui-ci avec des acides physiologiquement compatibles.

4. Utilisation selon la revendication 3, dans laquelle il s'agit pour les sels avec des acides physiologiquement compatibles du sel d'acide fumarique.
